Europäisches Patentamt

European Patent Office

Office européen des brevets

(11) Numéro de publication: **0 266 468**
**A1**

(12) **DEMANDE DE BREVET EUROPEEN**

(21) Numéro de dépôt: 86402479.9

(22) Date de dépôt: 06.11.86

(51) Int. Cl.⁴: **A61K 35/78** , A61K 31/20 , //(A61K35/78,31:355,31:59),(A6-1K31/20,31:355,31:59)

(43) Date de publication de la demande:
11.05.88 Bulletin 88/19

(84) Etats contractants désignés:
AT BE CH DE ES GB GR IT LI LU NL SE

(71) Demandeur: **Laboratoires Natura Medica, Société Anonyme dite:**
**R.N. 100**
**F-30390 Domazan(FR)**

(72) Inventeur: **Masse, Jean-Pierre**
**152 Rue de las Sorbes**
**F-34000 Montpellier(FR)**

(74) Mandataire: **Portal, Gérard et al**
**Cabinet Beau de Loménie 55, rue d'Amsterdam**
**F-75008 Paris(FR)**

(54) Composition thérapeutique comportant de l'acide alpha-linolénique et un composé susceptible de favoriser le passage de l'acide au travers de la membrane cellulaire et extrait de plantes comprenant l'acide et le composé et procédé de préparation de l'extrait.

(57) L'invention a pour objet une composition thérapeutique comportant à titre de principe actif de l'acide α-linolénique et un alcool d'origine végétale comme les tocophérols ou les sitosstérols. De préférence, la composition contient d'autres acides gras et est un extrait organique de plantes notamment de la famille des résédacées.

L'invention concerne également les extraits de plantes à titre de produit nouveau.

La composition thérapeutique est notamment utile comme anti-inflammatoire.

EP 0 266 468 A1

## Composition thérapeutique comportant de l'acide α-linolénique et un composé susceptible de favoriser le passage de l'acide au travers de la membrane cellulaire et extrait de plantes comprenant l'acide et le composé et procédé de préparation de l'extrait

L'invention concerne le domaine des médicaments, notamment anti-inflammatoires.

Elle a pour objet une composition thérapeutique caractérisée en ce qu'elle comporte, en tant que principe actif, au moins de l'acide α-linolénique et au moins un composé susceptible de favoriser le passage de l'acide au travers de la membrane cellulaire, en association avec un excipient physiologiquement acceptable.

Elle a également pour objet les extraits de plantes comportant le principe actif.

L'acide α-linolénique n'a pas d'activité thérapeutique seul.

Selon la présente invention, il a été trouvé que le principe actif décrit ci-dessus présentait des propriétés thérapeutiques, notamment anti-inflammatoires, parfaitement inattendues. Parmi les composés susceptibles de favoriser le passage de l'acide α-linolénique au travers de la membrane cellulaire, on peut citer les alcools d'origine végétale comme les tocophérols (α-, β-, γ-, δ-) ou les sitostérols comme le β-sitostérol. Outre ces deux types de produits, le principe actif peut comporter d'autres composés et, notamment, des acides gras en $C_{16}$ et $C_{18}$ comme l'acide palmitique, l'acide stéarique, l'acide oléique, l'acide linoléique. Cette présence résulte d'ailleurs souvent du procédé d'obtention du principe actif comme cela sera compris ci-après.

Ainsi, de préférence, le principe actif aura la composition suivante en pourcentage pondéral :

acide α-linolénique     10-80 et de préférence 15-60

composé     10-80 et de préférence 15-60

acide gras en $C_{16}$ et $C_{18}$     0-65 et de préférence 15-55
autres que l'acide α-linolénique

Le principe actif est de préférence un extrait aqueux ou organique de plantes qui renferment de l'acide α-linolénique et un composé susceptible de favoriser le passage de l'acide au travers de la membrane cellulaire.

On appelle extrait aqueux ou organique le produit qui résulte de l'action d'un solvant aqueux ou organique sur un broyat de plantes. Il s'agit donc de l'extraction des produits solubles ou miscibles dans ces solvants.

Parmi les plantes convenant pour la présente invention, on préférera celles de la famille des résédacées comme les réséda Lutéa, Luteola, Glauca L, suffruticosa ou alba, Jacquini Rehb, odorata, complicata bory, phyteuma.

On peut utiliser les plantes entières récoltées en fleurs ou en fruits ou des graines. On les sèche ensuite à l'abri de la lumière sous courant d'air.

L'extrait aqueux résulte avantageusement de l'épuisement par de l'eau d'un broyat de plantes ou de graines puis de la récupération par un moyen approprié du soluté sec.

Comme moyen approprié, on pourra utiliser la lyophilisation ou la nébulisation (après addition pour cette dernière d'un viscosifiant ou adsorbant tel que l'hydroxy-propyl-méthyl-cellulose (HPMC)), l'acide silicique.

On peut également extraire la solution aqueuse obtenue par un solvant organique puis concentrer la phase organique et terminer éventuellement par un fractionnement chromatographique de celle-ci. De manière avantageuse, le fractionnement sera tel qu'il permettra de récupérer les fractions contenant les acides gras. De manière préférée, l'extrait aqueux résultera d'un fractionnement chromatographique solide-liquide, l'éluant étant un mélange benzène-méthanol dans une proportion 50/50 et le support étant de la silice, l'élution étant effectuée par fraction de 10 ml et de la récupération des fractions comprises entre la quarante-troisième et la soixantième.

L'extrait organique résulte de l'épuisement par un solvant organique tel que l'éther, l'hexane, le méthanol ou du $CO_2$ liquide en phase supercritique d'un broyat de plantes ou de graines puis de la récupération par évaporation du soluté sec. Comme solvant, on préférera toutefois l'éther, l'hexane ou l'éther de pétrole et encore de préférence le méthyl-tertiobutyléther, ou le $CO_2$ liquide en phase supercritique à l'état pur ou éventuellement avec des traces d'eau ou d'éthanol ou similaire (pour extraire les composés plus ou moins polaires).

Il faut souligner que tous les extraits de plantes sont nouveaux et le déposant les revendique à titre de produits nouveaux caractérisés par leur procédé d'obtention comme il résulte de la description précédente.

Les compositions thérapeutiques selon l'invention se sont révélées utiles en tant qu'anti-inflammatoire-antihistaminique-antihémorroïdaire -antivariqueux -anticoagulant - vasoconstricteur ou antisporiasique.

La concentration de l'extrait dans le produit fini est avantageusement comprise entre 1O et 25% en poids pour l'extrait aqueux et 2 à 12 % pour l'extrait organique.

L'administration peut être effectuée par voie locale : pommade, crème, spray, lotion, gel ou par voie intradermique : ampoules ou à l'aide d'excipients classiques ou par voie générale orale ou parentérale : capsules, gouttes buvables, etc...

L'invention est maintenant illustrée par des exemples particuliers de réalisation non limitatifs.

Obtention des extraits

Exemple : Obtention de l'extrait aqueux

La plante est pulvérisée jusqu'à obtention d'une poudre assez grossière avec un broyeur type à galets. La poudre obtenue est épuisée par 8 fois son poids d'eau distillée à une température de 7O°C pendant 8 h sous agitation mécanique.

L'extrait obtenu après filtration et expression du marc est séché par nébulisation ou lyophilisation.

La nébulisation est effectuée après addition d'hydroxy-propyl-méthyl-cellulose (HPMC).

Exemple 2: Obtention de l'extrait aqueux (2e méthode)

L'extrait aqueux de l'exemple 1 est précipité avec de l'éthanol à 95°. On obtient ainsi un mélange de corps gras.

Exemple 3 : Obtention de l'extrait aqueux (3e méthode)

A partir de la phase aqueuse obtenue selon l'exemple 1, au lieu de lyophiliser ou nébuliser, on extrait le soluté par de l'éther dans une ampoule à décanter. On concentre sous pression réduite la phase éthérée jusqu'à l'obtention d'une huile verte. Cet extrait est fractionné ensuite sur colonne de silice avec comme éluant un mélange de benzène et de méthanol dans les proportions 5O/5O. On recueille des fractions de 1O ml. A partir de la 45e fraction, la solution ne contient plus que des acides gras, jusqu'à la soixantième. On réunit de 45 à 6O.

Exemple 4 : Obtention de l'extrait éthéré

L'opération a été réalisée sur la plante entière ou sur les graines .

-1 kg de plante est épuisé par macération sous agitation mécanique pendant 48 h à température ambiante par 6 l de solvant organique (éther, éther de pétrole et surtout hexane)

-Après filtration sur papier, la solution obtenue est concentrée sous pression réduite jusqu'à obtention d'un extrait huileux que l'on sèche sous courant d'azote.

L'extrait éthéré a la composition chimique suivante (en pourcentage pondéral), à 1O % d'erreur près:

-dl $\alpha$-tocophérol 18
-$\beta$-sitostérol 18
-acide palmitique 13,5
-acide stéarique 1
-acide oléique 8
-acide linoléique 1O
-acide $\alpha$-linolénique 31,5

NB : la conservation de cet extrait doit se faire à l'abri de la lumière et au frais.

Exemple 5 : Obtention de l'extrait alcoolisé

L'opération est réalisée dans les mêmes conditions que pour l'exemple 4, mais en prenant comme solvant le méthanol.

3

Propriétés pharmacologiques

L'activité anti-inflammatoire des extraits obtenus selon les exemples 1 à 5 a été évaluée par voie générale par comparaison avec un témoin chez le rat Wistar et avec l'indométacine anti-inflammatoire puissant par le pourcentage de diminution de l'enflure de la patte postérieure, provoquée par un agent phlogogène : la carragénine. Les résultats sont réunis dans le tableau I de la page suivante.

## TABLEAU I

Pourcentage de diminution de l'enflure après

|  | | 2 h | 3 h | 4 h | 5 h |
|---|---|---|---|---|---|
| Témoins | | 33,70 | 34,40 | 24,02 | 29,45 |
| Indométacine 5 mg/kg | | 38,60 | 71,62 | 40,42 | 32,8 |
| Exemple 1 | ① | 36 | 54,55 | 31,37 | - |
| 100 mg/kg | ② | 49,73 | 41,22 | 22,47 | 7,83 |
| Exemple 2 100mg/kg | | 42,83 | 41,42 | 27,40 | 12,40 |
| Exemple 3 100mg/kg | | 48,24 | 55,80 | 46,46 | 43,76 |
| Exemple 4 5 mg/kg | | 53,41 | 70 | 54,70 | 41,08 |
| Exemple 5 5mg/kg | | 24,37 | 16,17 | - | - |

① et ② correspondent à deux lots de rats différents.

## TOXICITE

### A/ Toxicité aiguë

Déterminé chez le rat mâle de souche Wistar et la souris mâle de souche Swiss selon deux voies d'administration : per os, intrapéritonéale.

$DL_{50}$ rat

per os : $DL_{50}$ supérieure à 5 g/kg

IP : $DL_{50}$ supérieure à 2 g/kg

$DL_{50}$ souris

per os : $DL_{50}$ supérieure à 5 g/kg

IP : $DL_{50}$ supérieure à 2 g/kg

### B/ Tolérance locale du nébulisat et de l'extrait éthéré sous forme de crème à 10%.

La tolérance locale de la crème a été réalisée chez le lapin Fauve de Bourgogne par administration cutanée réitérée de 2 g/kg de crème pendant 4 semaines sur le flanc de l'animal préalablement rasé sur une surface de 80 cm.

Le traitement n'a pas influencé l'évolution pondérale de nos animaux. La consommation de nourriture et d'eau de boisson a été identique chez les animaux témoins et animaux traités.

La repousse des poils s'est effectuée normalement.

L'examen des téguments a montré une peau lisse et extrêmement souple.

A l'autopsie, l'examen et la pesée des organes suivants : foie, rate, reins, testicules, surrénales, coeur, thyroïde, hypophyse n'ont pas révélé de signe de toxicité. Aucune différence n'est apparue entre animaux témoins et animaux traités.

L'examen minutieux de la muqueuse gastrique et duodénale n'a révélé aucune anomalie.

### Résultats cliniques

Les tests ont été effectués avec l'extrait de l'exemple 1 sous forme d'une crème à 10% en poids d'extrait organique.

Les résultats consignés dans le tableau II de la page suivante et relevés par des médecins sont très satisfaisants sur les hémorroïdes externes ou internes, accompagnées ou non de prurit ou de légères fissures, sur les varices, sur les piqûres d'insectes.

Le produit est en outre un excellent adoucissant de la peau.

TABLEAU II

| Sexe | Age | Diagnostic | Résultats | | Tolérance | Temps latence | Durée |
|------|-----|-----------|-----------|-----------|-----------|---------------|-------|
| | | | Médecin | Malade | | | |
| M | 36 | Thrombose avec oedème externe | B | B | B | 2 j | 15 j |
| M | 75 | Prurit anal | B | B | B | 2 | 8 |
| M | 78 | Anite chez un diarrhéique | Moyen | Moyen | B | 10 | 10 |
| M | 48 | Thrombose avec oedème | B | B | B | 1 | 8 |
| F | 43 | Crise hémorroïdaire | Non revue | | | | |
| M | 50 | Anite avec sang-douleur | B | B | B | 2 | 15 |
| F | 59 | Fissure super-ficielle-douloureuse | B | B | B | 5 | 15 |
| F | 75 | Hémorroïdes + mycose péri-anale | Moyen | Moyen | | | |
| F | 62 | Anite avec sang | B | B | B | 3 | 15 |

0 266 468

TABLEAU II (suite)

| Sexe | Age | Diagnostic | Résultats Médecin | Résultats Malade | Tolérance | Temps latence | Durée |
|------|-----|------------|-------------------|------------------|-----------|---------------|-------|
| F | 57 | Brûlures anales chroniques | B | B | B | Utilisation occasionnelle | |
| M | 36 | Hémorroïdes internes | B | B | B | Immédiat | 10 |
| M | 51 | Prurit anal + hémorroïdes | B | B | B | 2 | 15 |
| M | 36 | Anite + Prurit | Moyen | Moyen | | | |
| M | 49 | Inflammation hémorroïdaire + prurit post-opératoire | B | B | B | 2 | 8 |
| | | | B | B | B | 2 | 15 |
| F | 59 | Douleur + inflammation après sclérose | B | B | B | | 15 |

## Revendications

1. Composition thérapeutique, caractérisée en ce qu'elle comporte, en tant que principe actif, au moins de l'acide $\alpha$-linolénique et au moins un composé susceptible de favoriser le passage de l'acide au travers de la membrane cellulaire, en association avec un excipient physiologiquement acceptable.

2. Composition thérapeutique selon la revendication 1, caractérisée en ce que le composé est choisi parmi les tocophérols ou les sitostérols seuls ou en mélange.

3. Composition thérapeutique selon la revendication 1, caractérisée en ce qu'elle comporte d'autres acides gras en $C_{16}$ et $C_{18}$.

4. Composition thérapeutique selon l'une des revendications 1 à 3, caractérisée en ce que le principe actif comprend en pourcentage pondéral :

acide $\alpha$-linolénique    10 à 80

composé     1O à 8O

acides gras en $C_{16}$ et $C_{18}$     O à 65

autres que l'acide $\alpha$-linolénique

5. Composition thérapeutique selon la revendication 4, caractérisée en ce que le principe actif est un extrait aqueux ou organique de plantes qui renferment de l'acide $\alpha$-linolénique et un composé susceptible de favoriser le passage de l'acide au travers de la membrane cellulaire.

6. Composition thérapeutique selon la revendication 5, caractérisée en ce que les plantes sont de la famille des résédacées.

7. Composition thérapeutique selon l'une des revendications 5 ou 6, caractérisée en ce que l'extrait organique résulte de l'épuisement par un solvant organique d'un broyat de plantes ou de graines puis de la récupération, par évaporation, du soluté sec.

8. Extrait de plantes aqueux ou organique qui renferment de l'acide $\alpha$-linolénique et un composé susceptible de favoriser le passage de l'acide au travers de la membrane cellulaire.

9. Extrait de plantes selon la revendication 8, caractérisé en ce que les plantes sont de la famille des résédacées.

1O. Extrait de plantes selon l'une des revendications 8 ou 9, caractérisé en ce qu'il résulte de l'épuisement par un solvant organique d'un broyat de plantes (entières récoltées en fleurs et/ou en fruits) ou de graines puis de la récupération, par évaporation, du soluté sec.

11. Extrait de plantes aqueux ou organique selon la revendication 8, caractérisé en ce qu'il comporte en pourcentage pondéral :

acide $\alpha$-linolénique     1O à 8O

composé     1O à 8O

acides gras en $C_{16}$ et $C_{18}$     O à 65

autres que l'acide $\alpha$-linolénique

12. Procédé de préparation d'un extrait de plantes tel que défini selon la revendication 8, 9 ou 11, caractérisé en ce qu'on effectue un épuisement par de l'eau d'un broyat de plantes ou de graines de ces plantes suivi de la récupération par un moyen approprié du soluté sec, de préférence par lyophilisation ou nébulisation.

13. Procédé selon la revendication 12, caractérisé en ce qu'on extrait à nouveau l'extrait aqueux par un solvant organique, puis on concentre la phase organique et éventuellement on termine par un fractionnement chromatographique.

14. Procédé de préparation d'un extrait de plantes selon la revendication 8, 9, 1O ou 11, caractérisé en ce qu'on effectue un épuisement par un solvant organique d'un broyat de plantes ou de graines desdites plantes suivi de la récupération de l'extrait par évaporation du soluté sec.

15. Procédé selon la revendication 14, caractérisé en ce que le solvant organique est choisi parmi l'éther, l'hexane, le méthanol, le méthyl-tertiobutyléther et le $CO_2$ liquide en phase supercritique à l'état pur, ou éventuellement avec des traces d'eau ou d'éthanol.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| X | EP-A-0 115 419 (EFAMOL LTD)<br>* Revendications 1,4; page 10, lignes 23-25 *<br><br>--- | 1-3,5 | A 61 K 35/78<br>A 61 K 31/20 //<br>(A 61 K 35/78<br>A 61 K 31:355<br>A 61 K 31:59 ) |
| X | ROTE LISTE, 1961, page 1040, Editio Cantor, Aulendorf/Württ, DE<br>* Page 1040: "Vulno-Grandelat" *<br><br>--- | 1-3,5 | (A 61 K 31/20<br>A 61 K 31:355<br>A 61 K 31:59 ) |
| A | UNLISTED DRUGS, vol. 15, no. 1, janvier 1963, page 5, Chatham, New Jersey, US<br>* Page 5: "Domestol" *<br><br>----- | 1-3,5 | |

DOMAINES TECHNIQUES
RECHERCHES (Int. Cl.4)

A 61 K

Le présent rapport de recherche a été établi pour toutes les revendications

| Lieu de la recherche<br>LA HAYE | Date d'achèvement de la recherche<br>07-07-1987 | Examinateur<br>PEETERS J.C. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82